# EUROPEAN PATENT SPECIFICATION

(11) **EP 2 840 384 B1**
(45) Date of publication and mention of the grant of the patent: **17.06.2020**
(21) Application number: 13181050.9
(22) Date of filing: 20.08.2013
(51) Int. Cl.: G01N 21/87, B07C 5/36, B07C 5/342, G01N 33/38

(54) **Apparatus for the classification of colourless diamonds**
Vorrichtung zur Klassifizierung von farblosen Diamanten
Appareil pour la classification de diamants incolores

(43) Date of publication of application: 25.02.2015
(73) Proprietor: SATT Gems AG, 4051 Basel (CH)
(72) Inventor: Krzemnicki, Michael S., 4001 Basel (CH); Chalain, Jean-Pierre, 4001 Basel (CH); Steinacher, Michael, 4054 Basel (CH); Hänni, Henry A., 4102 Binningen (CH)
(74) Representative: BOVARD AG

(56) References cited:
- EP-A1- 1 657 544
- WO-A2-2012/172573
- GB-A- 2 267 147
- GB-A- 2 274 165
- US-A- 1 965 373
- US-A- 5 193 685

## Description

### Technical domain of the invention

The present invention relates to an apparatus for the separation of colourless untreated natural diamonds from colourless diamond imitations, colourless treated natural diamonds, colourless untreated synthetic diamonds and colourless treated synthetic diamonds comprising means for removing the colourless diamond imitations; and means for removing the colourless diamonds which are suspected to be treated natural diamonds, untreated synthetic diamonds or treated synthetic diamonds. For the purpose of the present invention, the term "a colourless diamond" is to be understood as comprising all diamonds which show no distinct hue when observed table up.

### Background of the invention

The watch and jewellery industry uses large quantities of polished colourless diamonds (often of small size) for their luxury products. These diamonds are natural, and have grown by a geologic process in nature. They are only cut and polished from rough material to be set into jewellery.

Natural diamonds may contain chemical impurities and are separated into two categories (so called type I and type II) and a number of subcategories (i.e. laA, laB, laAB, lb, Ila, lib) according to the presence (type I) or absence (type II) of nitrogen as a trace element detected by FT-infrared spectrometry.

Apart from natural diamonds, there are also synthetic diamonds on the market (also described as man-made or laboratory-created diamonds). There are mainly two methods to produce colourless synthetic diamonds: a) HPHT synthesis (High Pressure and High Temperature) and b) CVD synthesis (Chemical Vapour Deposition). Both methods may also be used for producing coloured synthetic diamonds.

The absence of colour in a natural or synthetic diamond can also be the result of a treatment. The most effective and sophisticated treatment is the so-called "HPHT" treatment (High Pressure and High Temperature), by which originally brownish (natural or synthetic) diamonds of type II or laB become colourless to near-colourless.

Beside this, there are many colourless to near-colourless diamond imitations on the market. They are mostly used in non-expensive fashion jewellery. A non-exhaustive list of colourless diamond imitations is: synthetic mois-sanite, cubic zirconium oxide (artificial product), glass (artificial product), synthetic spinel, and synthetic corundum.

There is a high interest in the industry to separate colourless natural diamonds of natural colour, i.e. colourless untreated natural diamonds, from the other kinds of diamonds (treated and/or synthetic) and the diamond imitations.

There exists a wide range of analytical approaches to separate colourless natural diamonds from the above-mentioned other products. These methods which are well documented in the scientific gemmological literature are generally described for "large" colourless diamonds (with a weight which is commonly greater than 0.15 carat, with a diameter for a round brilliant cut which is greater than 3.5 mm). They are mainly based on, but not limited to, visual observations, optical investigations such as measurement of the refractive index, measurement of the specific gravity, and most importantly, spectroscopic or spectrometric or magnetic characterisations.

There are two main challenges for determining the authenticity of small and colourless diamonds (the mean diameter of a round polished small diamond is smaller than about 3.5 mm).

The first is due to their small size, which makes not only the testing itself a challenge, but also may lead to a testing fee similar to the value of a small diamond (thus economically not justifiable).

The second challenge is that small diamonds are usually marketed in lots of large quantities (e.g. several thousands of single diamonds), thus multiplying the time and costs extensively.

The international patent application published under number WO 96/07894 discloses a method of distinguishing natural colourless diamonds from synthetic colourless diamonds, comprising a step of irradiating the diamond with ultraviolet light of a wavelength below about 250 nm and observing the induced phosphorescence decay. An apparatus is also disclosed, which allows an operator to observe the variation with time of the phosphorescence intensity and classify the diamond.

Such method and apparatus are only intended for natural and synthetic diamonds and not for diamond imitations. In addition, they do not allow an automatic and continuous classification of an elevated number of colourless diamonds.

As a result even nowadays the separation of colourless diamonds is still performed manually.

Regarding its transparency to UV short waves (UVc), a colourless diamond may be either transparent or not.

Type IIa, type IIb and pure type laB diamonds are transparent to UVc. Pure type laA and type laAB diamonds are generally not transparent to UVc and a slight UVc transparency may be noticeable if their nitrogen concentration is unusually low (e.g. below 4ppm).

On one hand, approximately 97% of colourless untreated natural diamonds are not transparent to UVc.

On the other hand, only a remaining small percentage (approximately 3%) of colourless untreated natural diamonds are transparent to UVc. Additionally, all colourless HPHT treated natural diamonds and all colourless synthetic diamonds, either HPHT or CVD grown, either HPHT treated or not, are also transparent to UVc.

Part of the pertinence of the invention relies on separating colourless diamonds that are not transparent to UVc from those that are transparent to UVc, therefore forming a first group of colourless diamonds not transparent to UVc and a second group of colourless diamonds that are transparent to UVc.

The first group will exclusively contain colourless untreated natural diamonds while the second group will contain a small quantity (3% as opposed to 97%) of colourless untreated natural diamonds plus, if present, all colourless HPHT treated natural diamonds plus, if present, all colourless synthetic diamonds, HPHT or CVD grown, HPHT treated or not.

The separation of this two groups is made automatically for small colourless diamonds, as is the separation between diamond and diamond imitation.

WO 2012/172573 A2 discloses a system and a method for segregating diamonds. GB 2 274 165 A deals with a method and apparatus for examining an object. EP 1 657 544 A1 discloses a method for discerning colourless and almost colourless diamonds.

### Summary of the invention

The main object of the invention is therefore to provide a reliable system which allows to separate automatically, rapidly, easily and reliably colourless untreated natural diamonds from colourless diamond imitations, colourless treated natural diamonds, colourless untreated synthetic diamonds and colourless treated synthetic diamonds. As already mentioned above, the term "a colourless diamond" in the present invention refers to all diamonds which show no distinct hue when observed table up. In addition, this system alsodetermines the authenticity of diamonds of a small size, that is, diamonds that are round and polished and have a mean diameter smaller than about 3.5 mm.

This object is achieved by an apparatus according to claim 1.

### Brief description of the drawings

Other features and advantages of the invention will now be described in detail in the following disclosure which is given with reference to the appended figures which schematically represent:
Fig. 1: an apparatus according to the invention;
Fig. 2: a detail view of the apparatus of Fig. 1;
Fig. 3: a diamond stone correctly positioned and irradiated during the RSD detection;
Fig. 4: equipment for the RSD;
Fig. 5a-d: RSD results;
Fig. 6: a diamond stone correctly positioned and irradiated during an UVc transmission measurement;
Fig. 7: equipment for the UTM;
Fig. 8a-f: UVc transmission measurement results together with the respective IR absorption spectra; and
Fig. 9a-b: an example of a flow sheet.

### Detailed disclosure of the invention

A preferred embodiment of the invention will now be described hereinafter with reference to the accompanying drawings.

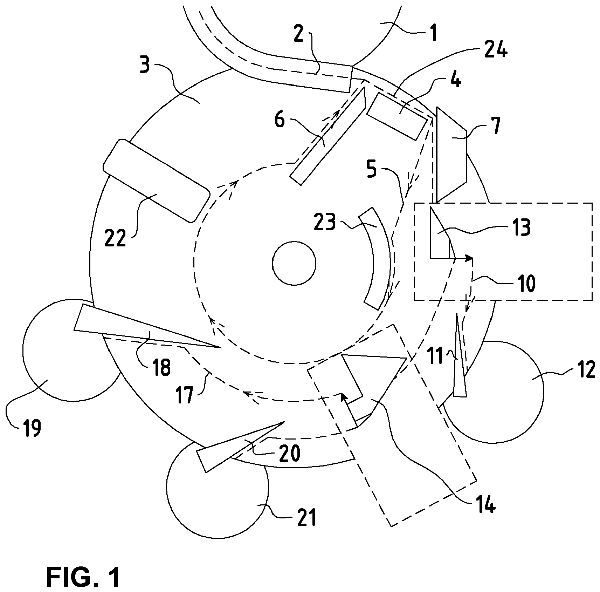

As can be seen in Fig. 1, the starting product, usually a batch of polished small stones, which are colourless and composed of untreated or treated natural or synthetic diamond or of a diamond imitating material, is introduced into a vibrating bowl 1 equipped with a feeder 2. The stones reaching the top of the vibrating bowl 1 are pushed to feeder 2 and brought on a table which is preferably a glass disk 3 rotated by a generally electric motor (not represented).

The rotation of disk 3 leads the stones one by one to a set of orientation chicanes 4 which deviates the stones that are not correctly oriented to an inner orbit 5 for reaching a chicane 6 which sends them back to the set of orientation chicanes 4.

The rotation of disk 3 brings the correctly oriented stones to an aligning and spacing device 7 which ensures that the stones are spaced one from another by a determined clearance, for example 12 mm.

The stones then face Raman detection means 13 where their Raman shift is measured in a wavelength range of between 565 nm and 575 nm. In particular, their Raman shift (emission) signal is observed at a wavelength of 572.6 nm, which is the characteristic Raman shift wavelength Wₑ of natural or synthetic diamond when using an excitation wavelength of 532 nm.

Advantageously, a reference signal may also be observed at a different wavelength, for example between 555 and 565 nm. The use of two such signals allows to reduce background signals and therefore improves the measurement results.

When the intensity of the calculated signal of a stone at the predetermined wavelength Wₑ is lower than a predetermined value V_{e'} used as a discard threshold, it is considered that the stone is a diamond imitation and must be removed.

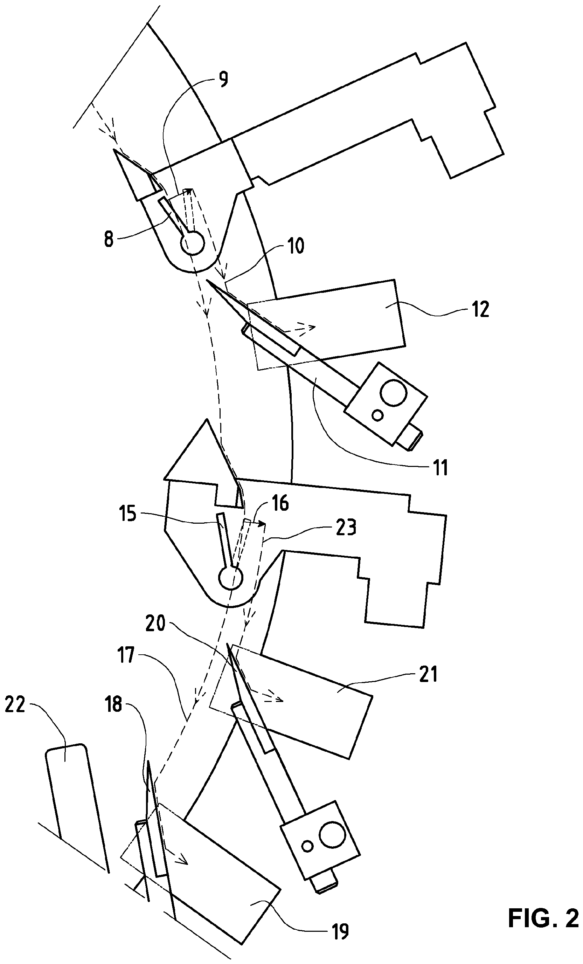

Represented in Fig. 2 is a discard removing arm 8 aimed at rotating in a direction denoted by arrow 9, in order to push a diamond imitation stone into a partial trajectory 10 having a larger diameter and reaching a discard chicane 11 which leads the diamond imitation to a vessel 12 for diamond imitation stones.

Turning back to Fig. 1, one can see that the remaining stones, now diamonds, are brought by the further rotation of disk 3 to UVc transmission measurement means 14, where their transmission signal is observed in a predetermined wavelength region Rₐ, in particular in the UVc region, for example 270 nm.

When the intensity of the absorbance signal of a stone in the predetermined wavelength region Rₐ is lower than a predetermined value Vₐ used as a re-examination threshold, it is considered that the stone is questionable and must be re-examined, with the same and/or other methods. The questionable stone is either a HPHT treated natural diamond, a synthetic diamond (either treated or untreated) or a rare untreated natural diamond - approximately 3% of untreated natural diamonds are concerned.

Represented in Fig. 2 is a re-examination removing arm 15 aimed at rotating in a direction denoted by arrow 16, in order to push a questionable stone into an outer orbit 23 and reaching a re-examination chicane 20 which leads the questionable stone to a vessel 21 for stones that deserve further investigation.

The stones which were not pushed by the removing arm 8 nor by re-examination removing arm 15 are brought by the further rotation of disk 3 on track 17 to a successful stone chicane 18 which leads them to a vessel 19 for colourless to near-colourless untreated natural diamond stones.

Optionally, a set of cameras (not shown on Fig. 2) may be added before chicane 18, to measure any diamonds on orbit 17, for their depth, diameter, crown, pavilion, etc. and a computer will calculate the desired cut proportion values.

Preferably a fixed disk cleaner 22 is provided, for example between re-examination chicane 18 and return chicane 6.

### Complements to the apparatus according to the invention

It goes without saying that various numerous additional appropriate means may be added, for example, means for cleaning the stones, means for weighting them, means for detecting the presence of a diamond or diamond imitation in a specific location in front of the Raman or UVc sensors, electronic means for controlling the measurements and their sequence, memory means, recording means for recording the measurement results, processing means for controlling the movement of the arms, analysing means for taking a decision on the results (namely by way of comparison with threshold values), means for counting the stones, statistic means, means for signalling errors, means for emitting a noise or turning on a light or other device in order to advise an operator of a result, an error, etc.

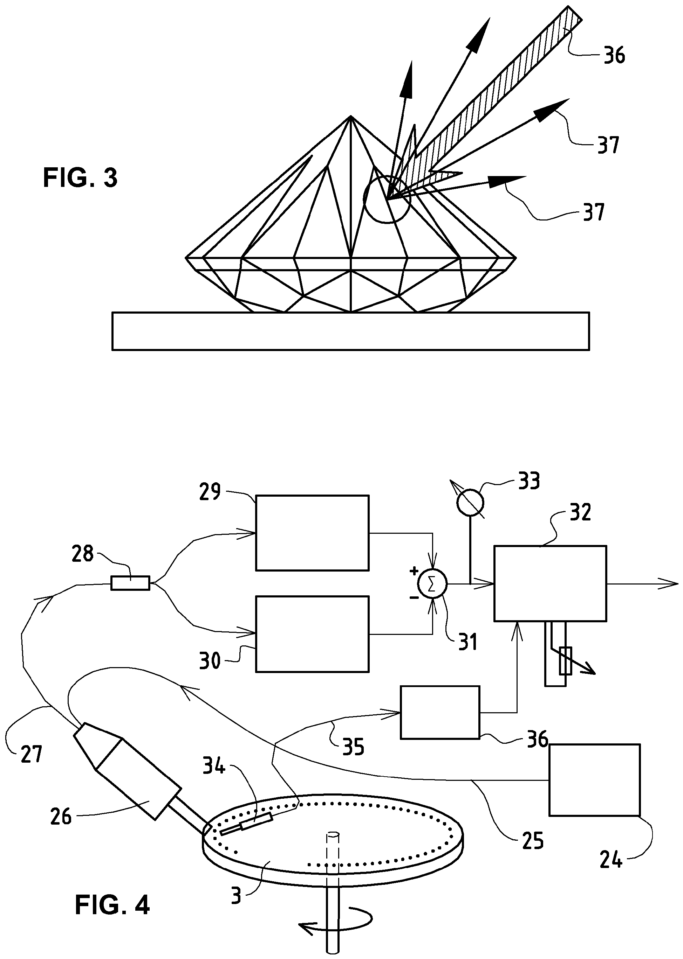

These means are known per se and need not be further detailed herein. Therefore, only a few of them will be described hereinafter with reference to the appended figures 3 to 8.

### Example and details of a Raman Shift Detection (RSD)

Fig. 3 represents a polished round brilliant cut diamond correctly positioned on disk 3, hit by a 100 mW focused green laser 36 (532 nm) onto the pavilion of the diamond and emitting a characteristic diamond Raman peak 37 at 572.6 nm.

Represented in Fig. 4 is the rotating disk 3 with a 532 nm laser source 24 connected by optic fibre cable 25 to a Raman probe 26 aimed at irradiating a stone, preferably with an irradiating angle of 90 degrees with the pavilion of the polished stone. The signal of the scattered light received by the Raman probe 26 is conducted by an optic fibre cable 27 to a fibre optic splitter 28 and then both to a Raman channel (565-575 nm) photo-detector 29 and to a background channel (555-565 nm) photo-detector 30. Both signals are band-passed, filtered, pre-amplified and then substracted 31. The resulting signal is then coupled with a stone at place signal. The coupled signals are finally processed and compared to a suitable threshold value in a processing means 32.

The stone at place signal is provided to detect when a stone is at the correct position on disk 3 for the Raman measurement. The information is transmitted by an optic fiber cable 35, treated by sensor electronic means 36 and forwarded to signal processing means 32.

The inspection is passed both when the stone is correctly placed on disk 3 in front of Raman probe 26 and the Raman signal exceeds a given threshold.

However, if the Raman signal does not reach the threshold value, arm 8 (Fig. 2) then moves and pushes the stone into a larger orbit 10 (Fig.2) so that it a chicane 11 (Fig. 2) drives it into the re-examination vessel 12 (Fig 2).

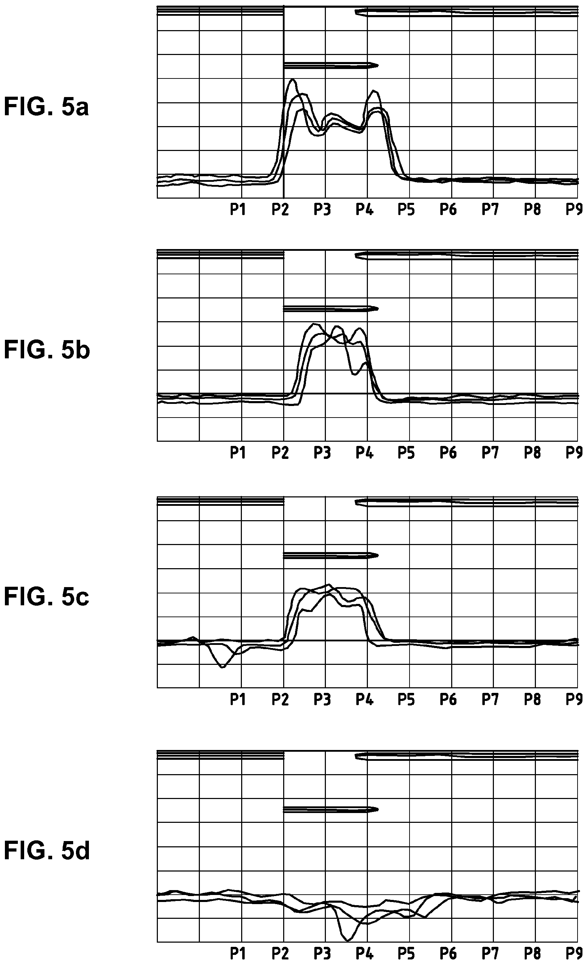

Fig. 5a-d are the results (oscilloscope diagrams) of measurements performed at a disk rotation speed of 6 rpm with a glass disk with a diameter of 20 cm, with a laser current of 800 mA. The upper trace shows the digital signal from stone at place sensor 34. The lower trace displays the Raman signal output (Raman channel minus Background channel). Each diagram shows the maximum, mean and minimum measured values of ten runs (individual measurements) taken with the same sample, but with random rotational orientation of the stone. For Fig. 5a, it is abvious that each trace shows three different maximum peak intensities illustrating the rotation of the pavillion during each measurement.

Fig. 5a corresponds to a pure type laA diamond (d = 1.4 mm) having a "normal" nitrogen concentration. The Raman signal is high, 500 mV, i.e. above the threshold value. The stone therefore passes the test.

Fig. 5b corresponds to a diamond type laAB (d = 0.9 mm) having a "high" nitrogen concentration. The Raman signal is also high, 450 mV, i.e. above the threshold value. The stone therefore passes the test.

Fig. 5c corresponds to a diamond type IIa (d = 0.9 mm) having a "low" nitrogen concentration (not detectable by Fourier Transform Infrared spectroscopy FTIR). However, the Raman signal is still 400 mV, i.e. above the threshold value and thus the stone passes the test.

Fig. 5d corresponds to a cubic zirconia stone (d = 1.8 mm), i.e. a diamond imitation. No Raman signal is observed. Therefore the stone does not pass the test, and is pushed away by arm 8 (Fig. 2).

All these measurements show that the apparatus according to the invention can reliably detect a diamond indepently of its type and a diamond imitation by means of the Raman Shift Detection (RSD) and that a colourless diamond whichever is its type will pass the RSD test and therefore will continue its route on the glass plate while a diamond imitation will not pass the test and therefore will be sorted out into vessel 12 for further identification tests (e.g. Raman spectrometry).

### Example and details of a UVc Transmission Measurement (UTM)

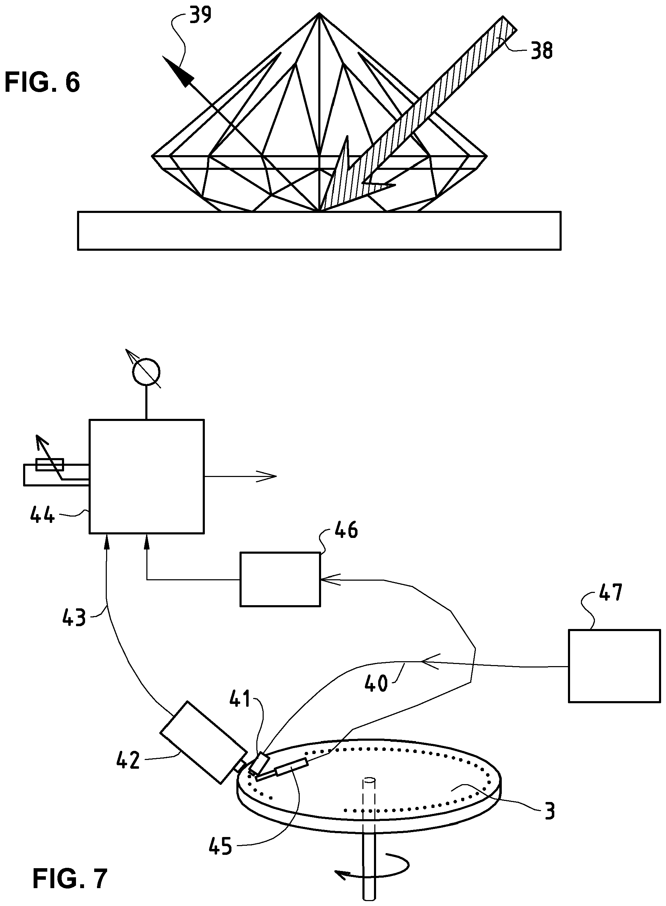

Fig. 6 represents a diamond stone correctly positioned on disk 3, hit by a 270 nm UVc light source 38 and transmitting a signal 39 from the internal reflection on the table (bottom) of the stone to an UVc detector.

Represented in Fig. 7 is the rotating disk 3 with a monitored UVc source 47 connected by glass fibre cable 40 to a UVc focusing lense 41 aimed at irradiating a stone, preferably with an irradiating angle of 90 degrees with reference to the pavillion's stone hit by the light rays. On the opposite side of the stone, the transmitted light coming from the internal reflection at the bottom of the diamond (i.e. the inside surface of the diamond's table) passes through a diffuser, a UVc filter and is measured by a UVc sensitive photo-detector 42. The signal of which is then amplified and conducted by a multipolar cable 43 to signal processing means 44.

On the other hand an optic sensor 45 detects when a stone is at the correct position on disk 3 for the UVc transmission measurement. The information is transmitted to sensor electronic means 46 and forwarded to signal processing means 44.

The inspection is passed both when the stone is correctly placed on disk 3 in front of UVc focusing lense 41 and the the transmission value V_{T} signal does not exceed a given threshold value.

However if the UVc transmission signal reaches the threshold value, arm 15 (Fig. 2) then deviates the diamond into an outer orbit so that chicane 20 drives it into the re-examination vessel 21.

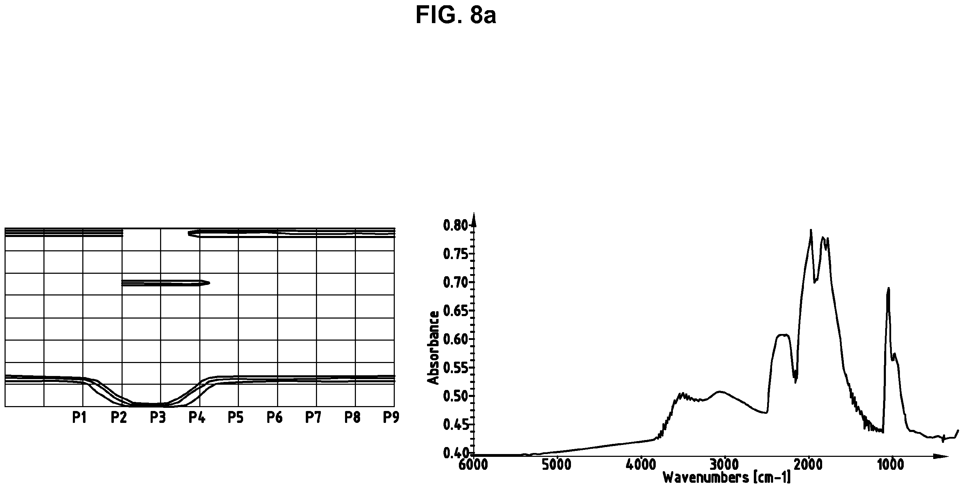

Fig. 8a-f are the results (oscilloscope diagrams) of measurements performed at a disk rotation speed of 6 rpm, with a UVc-LED current of 5 mA. Each diagram shows the maximum, mean and minimum measured values of ten to fifteen runs (individual measurements) taken with the same diamond, but with random rotational orientation of the stone. The upper trace shows the digital signal from the stone at place sensor 45. The lower traces display the preamplifier output signals from the UVc detector.

Fig. 8a corresponds to a pure type laA diamond (d = 1.4 mm) having a "normal" nitrogen concentration (independently determined by FTIR spectroscopy). The resulting signals indicate that no UVc transparency is detected. The stone therefore passes the test.

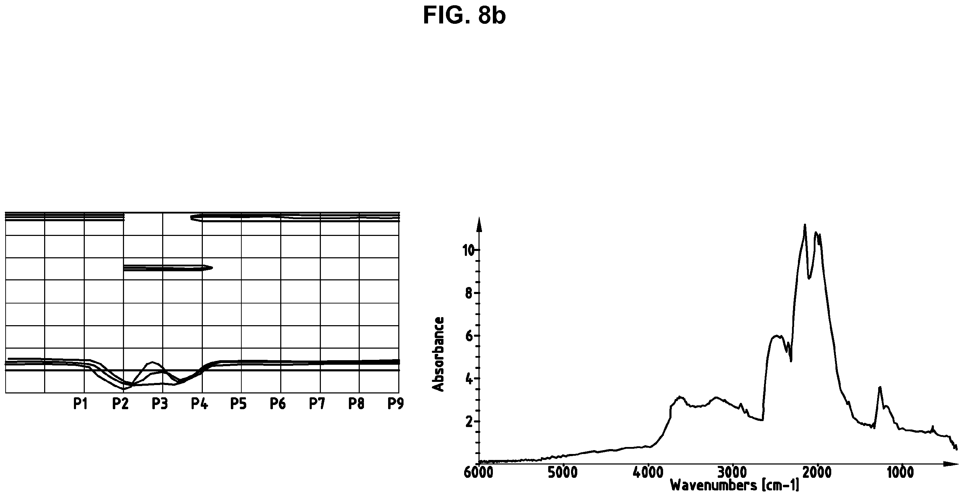

Fig. 8b corresponds to a type laAB diamond (d = 1.3 mm) having a "low" nitrogen concentration (independently determined by FTIR spectroscopy). The resulting signals indicate that depending on the stone orientation, no UVc transparency or a slight UVc transparency is detected. The signals being below a predetermined threshold, the diamond therefore passes the test.

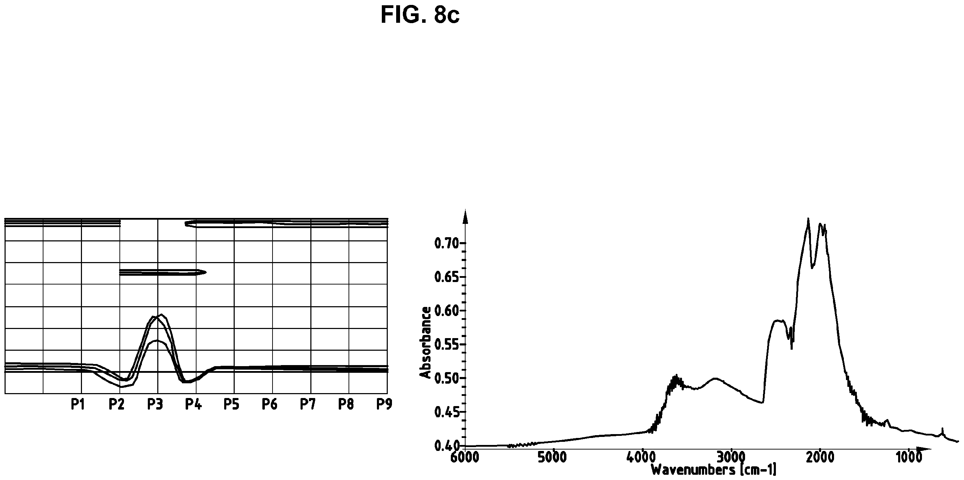

Fig. 8c corresponds to a diamond of the type laA (d = 1.4 mm) having a "very low" nitrogen concentration (independently determined by FTIR spectroscopy). The resulting signals indicate that depending on the stone orientation, a variable but significant UVc transparency is detected. The signals being over a predetermined threshold, the diamond therefore does not pass the test.

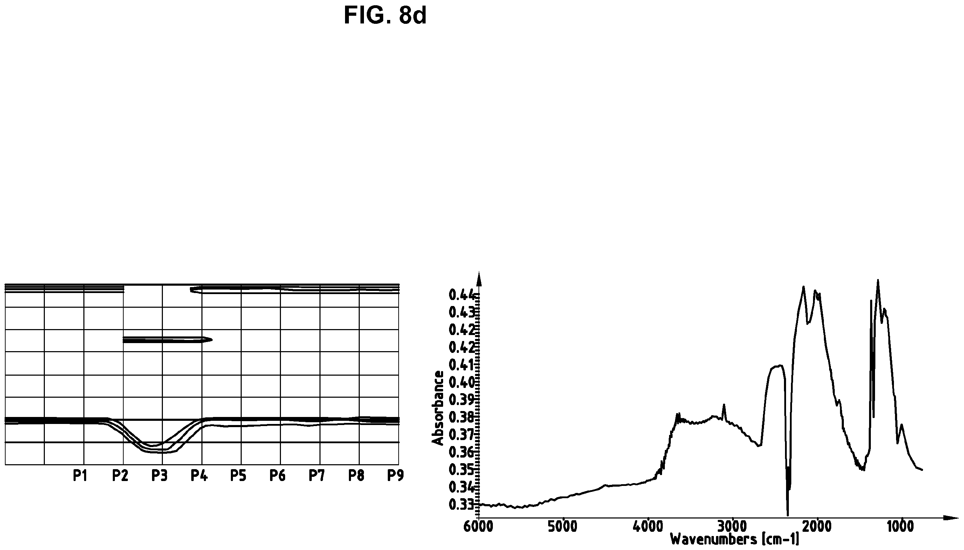

Fig. 8d corresponds to a type laAB, a diamond (d = 0.9 mm) having a "high" nitrogen concentration (independently determined by FTIR spectroscopy). The resulting signals indicate that no UVc transparency is detected. The signals being below a predetermined threshold, the diamond therefore passes the test.

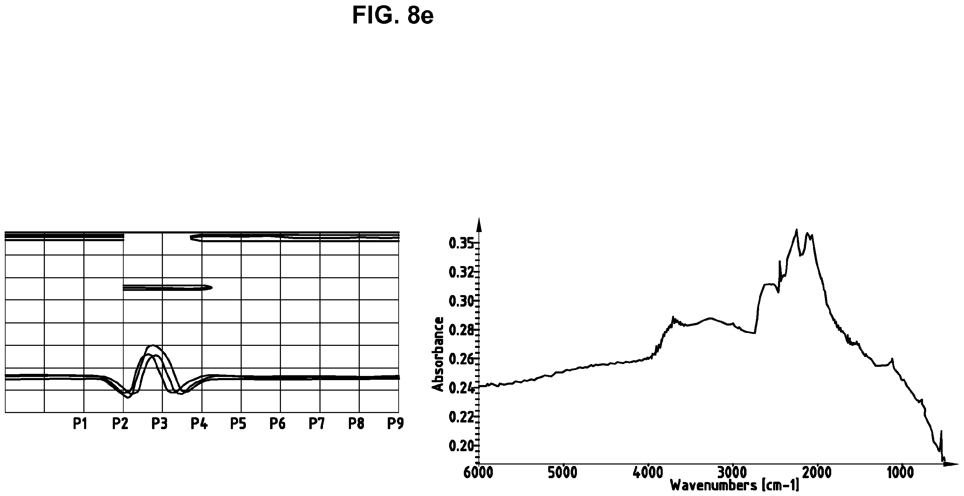

Fig. 8e corresponds to a type IIa (independently determined by FTIR spectroscopy) diamond (d = 0.9 mm). The resulting signals indicate that depending on the stone orientation, a variable but significant UVc transparency is detected. The signals being over a predetermined threshold, the diamond therefore does not pass the test.

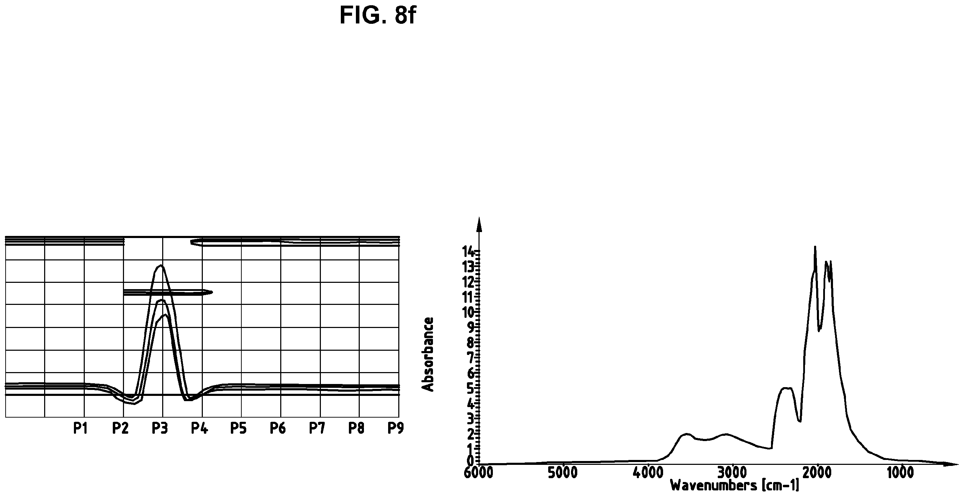

Fig. 8f corresponds to a type IIa (independently determined by FTIR spectroscopy) diamond (d = 1.1 mm). The resulting signals indicate that depending on the stone orientation, a variable but significant UVc transparency is detected. The signals being over a predetermined threshold, the diamond therefore does not pass the test.

All these measurements show that the apparatus according to the invention can reliably detect, based on their UVc transparency, type II colourless diamonds (potentially synthetic diamonds) and type IIa or type laB colourless diamonds (potentially HPHT treated natural diamonds) by means of this UVc transmission measurement and that colourless diamonds with very low nitrogen concentration will not pass the UVc transmission test and therefore will be sorted out into the vessel 21 for further testings (e.g. FTIR absorption spectrometry or low temperature photoluminescence).

### Flow sheet

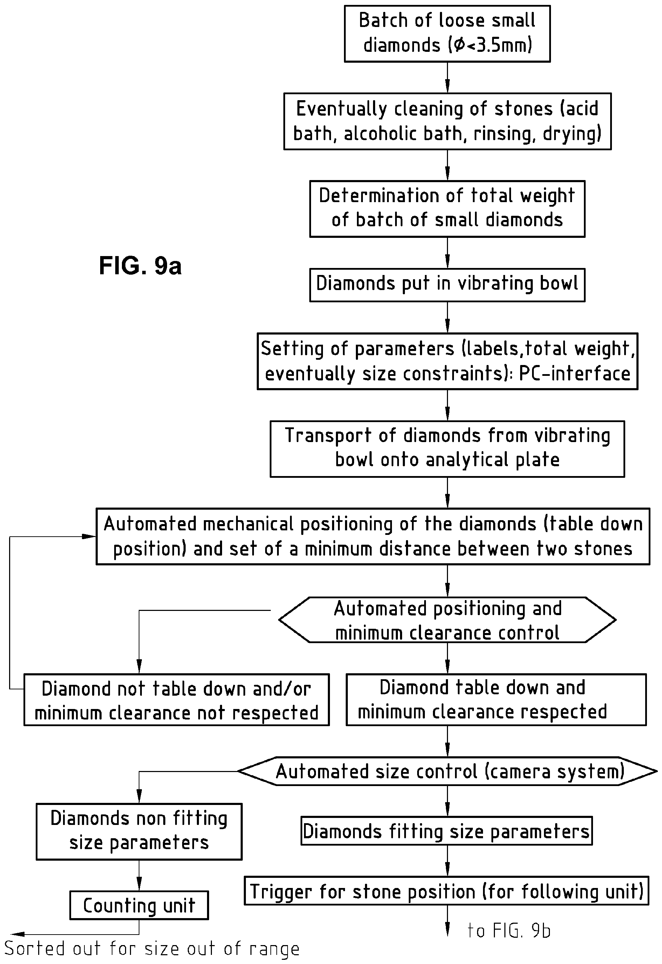

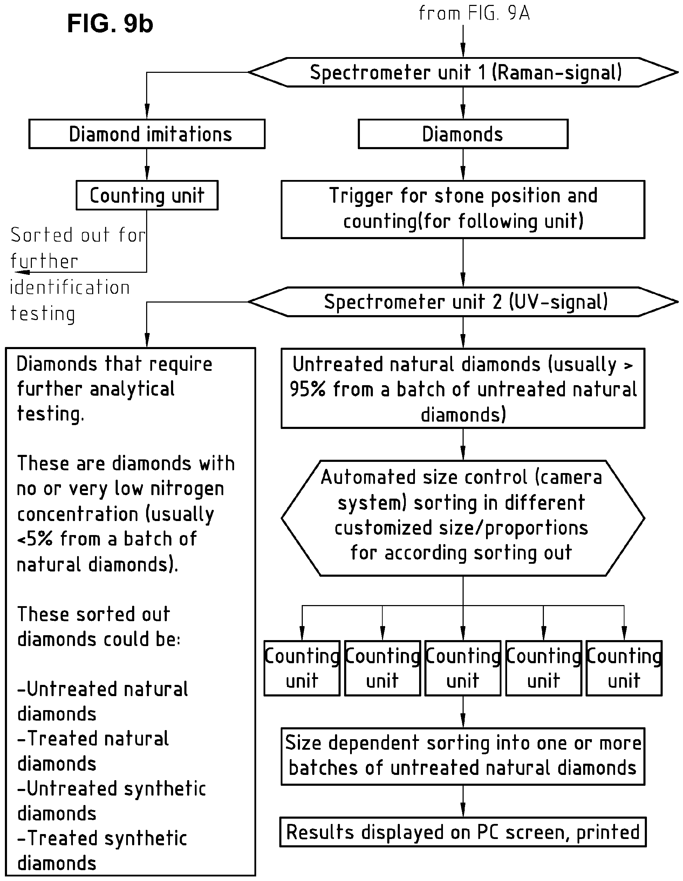

Fig. 9a-b is an example of a flow sheet for controlling the apparatus according to the invention.

## Claims

1. Apparatus for the automated separation of small colourless untreated natural diamonds from:
a) colourless diamond imitations;
b) colourless treated natural diamonds;
c) colourless untreated synthetic diamonds and
d) colourless treated synthetic diamonds;
wherein the small diamonds are round and polished and have a mean diameter smaller than about 3.5 mm; comprising:
supporting means (3) for supporting diamonds or diamond imitations, wherein said supporting means (3) is a disk;
feeding means (1,2) for bringing diamonds or diamond imitations on said supporting means (3);
moving means for moving said supporting means (3), wherein the moving means is a rotating means for rotating the disk;
detection means (13) for submitting colourless diamonds or diamond imitations to a Raman Shift Detection (RSD),
first removing means (8) for removing the colourless diamonds or diamond imitations having, at a predetermined wavelength (Wₑ), an emission signal, the intensity of which is lower than a predetermined value (Vₑ);
measurement means (14) for carrying out a UVc transmission measurement on the remaining colourless diamonds,
second removing means (15) for removing the colourless diamonds having, in a predetermined wavelength region (Rₐ), a transmission value (V_{T}) higher than a predetermined value (V_{T'}), and
means (20,21) for recovering the colourless untreated natural diamonds.

2. Apparatus according to claim 1, wherein said predetermined wavelength (Wₑ) is about 573 nm.

3. Apparatus according to claim 1 or claim 2, wherein said predetermined wavelength region (Rₐ) ranges from 200 to 280 nm.

4. Apparatus according to any one of the claims 1 to 3, further comprising means (4,6,23) for checking the correct position of said colourless diamonds or diamond imitations on said supporting means (3) and for returning wrongly positioned colourless diamonds or diamond imitations to said feeding means (1,2).

5. Apparatus according to any one of claims 1 to 4, further comprising means (7) for aligning and spacing said diamonds or diamond imitations with respect to one another by a predetermined clearance before the RSD detection means (13).

6. Apparatus according to claim 1, wherein said first removing means (8) and said second removing means (15) are able to modify the diameter of the respective trajectory of said diamonds or diamond imitations on said disk (3).

7. Apparatus according to any one of claims 1 to 6, further comprising means (22) for cleaning said supporting means (3).

## Patentansprüche

1. Vorrichtung zur automatisierten Trennung von kleinen farblosen unbehandelten natürlichen Diamanten von:
a) Farblosen Diamantimitationen;
b) Farblosen behandelten natürlichen Diamanten;
c) Farblosen unbehandelten synthetischen Diamanten und
d) Farblosen behandelten synthetischen Diamanten;
wobei die kleinen Diamanten rund und poliert sind und einen mittleren Durchmesser von kleiner als etwa 3.5 mm aufweisen;
umfassend:
Trägermittel (3) zum Tragen von Diamanten und Diamantimitationen, wobei das Trägermittel (3) eine Scheibe ist;
Zuführmittel (1, 2), um Diamanten oder Diamantimitationen auf das Trägermittel (3) zu bringen;
Bewegungsmittel, um das Trägermittel (3) zu bewegen, wobei die Bewegungsmittel Drehmittel sind, um die Scheibe zu drehen;
Detektionsmittel (13), um farblose Diamanten oder Diamantimitationen einer Raman Shift Detektion (RSD) zu unterwerfen,
erste Entfernungsmittel (8), um die farblosen Diamanten oder Diamantenimitationen zu entfernen, welche bei einer vorgegebenen Wellenlänge (Wₑ) ein Emissionssignal haben, dessen Intensität niedriger ist als ein vorgegebener Wert (Vₑ);
Messmittel (14), um eine UVc Transmissions-Messung an den verbleibenden farblosen Diamanten durchzuführen,
zweite Entfernungsmittel (15) zum Entfernen farbloser Diamanten, welche in einem vorgegebenen Wellenlängenbereich (Rₐ) einen Transmissionswert (V_{T}) höher als ein vorgegebener Wert (V_{T'}) aufweisen, und
Mittel (20, 21) zum Einsammeln der farblosen unbehandelten natürlichen Diamanten.

2. Vorrichtung nach Anspruch 1, wobei die vorgegebene Wellenlänge (Wₑ) in etwa 573 nm beträgt.

3. Vorrichtung nach Anspruch 1 oder Anspruch 2, wobei der vorgegebene Wellenlängenbereich (Rₐ) zwischen 200 und 280 nm liegt.

4. Vorrichtung nach einem der Ansprüche 1 bis 3, ferner umfassend Mittel (4, 6, 23) zum Prüfen der korrekten Position der farblosen Diamanten oder Diamantimitationen auf dem Trägermittel (3) und zum Rückführen falsch positionierter Diamanten oder Diamantimitationen zu den Zuführmitteln (1, 2).

5. Vorrichtung nach einem der Ansprüche 1 bis 4, ferner umfassend Mittel (7) zum Aufreihen und Anordnen von Diamanten oder Diamantimitationen in Bezug zueinander mit einem vorgegebenen Abstand vor den RSD Detektionsmitteln (13).

6. Vorrichtung nach Anspruch 1, wobei das erste Entfernungsmittel (8) und das zweite Entfernungsmittel (15) eingerichtet sind, um den Durchmesser der jeweiligen Bahn der Diamanten oder Diamantimitationen auf der Scheibe (3) zu modifizieren.

7. Vorrichtung nach einem der Ansprüche 1 bis 6, ferner umfassend Mittel (22) zum Reinigen des Trägermittels (3).

## Revendications

1. Appareil pour la séparation automatisée de petits diamants naturels non traités sans couleur vis-à-vis:
a) d'imitations de diamants incolores ;
b) de diamants naturels traités incolores ;
c) de diamants synthétiques non traités incolores et
d) de diamants synthétiques traités incolores ;
les petits diamants étant ronds et polis et possédant un diamètre moyen inférieur à 3.5 mm ;
l'appareil comprenant :
un moyen de support (3) pour porter des diamants et des imitations de diamants, le moyen de support (3) étant un disque ;
un moyen d'alimentation (1, 2) pour mener les diamants ou les imitations de diamants sur ledit moyen de support (3) ;
un moyen de déplacement pour déplacer ledit moyen de support (3), le moyen de déplacement étant un moyen d'actionnement en rotation pour faire tourner le disque ;
un moyen de détection (13) pour soumettre des diamants incolores ou des imitations de diamants à une détection du type Raman Shift Détection (RSD),
un premier moyen d'enlèvement (8) pour retirer les diamants incolores ou les imitations de diamants ayant, à une longueur d'onde prédéterminée (Wₑ), un signal d'émission, dont l'intensité est plus basse qu'une valeur prédéterminée (Vₑ) ;
un moyen de mesure (14) pour réaliser une mesure de transmission UVc sur les diamants incolores restants,
un deuxième moyen d'enlèvement (14) pour retirer les diamants incolores ayant, pour une plage de longueur d'onde prédéterminée (Rₐ), une valeur de transmission plus haute qu'une valeur prédéterminée (V_{T'}), et
des moyens (20, 21) pour récupérer les diamants naturels non traités incolores.

2. Appareil selon la revendication 1, dans lequel ladite longueur d'onde (Wₑ) prédéterminée est d'environ 573 nm.

3. Appareil selon la revendication 1 ou 2, dans lequel la plage de longueur d'onde prédéterminée (Rₐ) s'étend entre 200 et 280 nm.

4. Appareil selon l'une des revendications 1 à 3, comprenant en outre des moyens (4, 6, 23) pour contrôler la position correcte desdits diamants incolores ou des imitations de diamants sur ledit moyen de support (3), et pour renvoyer les diamants incolores ou les imitations de diamants mal positionnés audit moyen d'alimentation (1, 2).

5. Appareil selon l'une des revendications 1 à 4, comprenant en outre un moyen (7) pour aligner et espacer lesdits diamants ou imitations de diamants les uns par rapport aux autres selon un espacement prédéterminé avant le moyen de détection RSD (13).

6. Appareil selon la revendication 1, dans lequel ledit premier moyen d'enlèvement (8) et ledit deuxième moyen d'enlèvement (15) peuvent modifier le diamètre de la trajectoire respective desdits diamants ou des imitations de diamants sur ledit disque (3).

7. Appareil selon l'une des revendications 1 à 6, comprenant en outre un moyen (22) pour nettoyer ledit moyen de support (3).
